## Europäisches Patentamt
## European Patent Office
## Office européen des brevets

(19)

(11) Veröffentlichungsnummer: **0 242 836**
**A1**

(12) **EUROPÄISCHE PATENTANMELDUNG**

(21) Anmeldenummer: **87105806.1**

(22) Anmeldetag: **21.04.87**

(51) Int. Cl.³: **C 12 N 15/00**
**A 61 K 37/54, C 12 N 9/64**

(30) Priorität: **21.04.86 DE 3613402**
**17.12.86 DE 3643158**

(43) Veröffentlichungstag der Anmeldung:
**28.10.87 Patentblatt 87/44**

(84) Benannte Vertragsstaaten:
**AT BE CH DE ES FR GB GR IT LI LU NL SE**

(71) Anmelder: **BOEHRINGER MANNHEIM GMBH**
**Patentabteilung, Abt. E Sandhofer Strasse 112-132**
**Postfach 31 01 20**
**D-6800 Mannheim 31 Waldhof(DE)**

(72) Erfinder: **Mattes, Ralf, Dr. rer. nat.**
**Eyacher Strasse 37**
**D-8125 Oberhausen / Obb.(DE)**

(74) Vertreter: **Huber, Bernhard, Dipl.-Chem. et al,**
**Patentanwälte H. Weickmann, Dr. K. Fincke F.A.**
**Weickmann, B. Huber Dr. H. Liska, Dr. J. Prechtel**
**Möhlstrasse 22 Postfach 860 820**
**D-8000 München 86(DE)**

(54) **Gewebs-Plasminogen-Aktivator (tPA) - Derivat und seine Herstellung.**

(57) Ein neues Gewebs-Plasminogen-Aktivator (tPA) - Derivat, weist eine Aminosäuresequenz auf, die der des natürlichen tPA entspricht, in der jedoch die die EGF-Domäne bildenden Aminosäuren des natürlichen tPA-Moleküls fehlen und mindestens einer der Kringel I oder II, die Verbindungsregion, die katalytische Region und der Fibrinfinger vorhanden sind; insbesondere können zusätzlich die daran anschließenden Aminosäuren bis mindestens zur ersten Disulfidbrücke der Domäne des Kringels I oder die gesamten Aminosäuren der Domäne des Kringels I bis zur ersten Disulfidbrücke der Domäne des Kringels II oder Kringel II fehlen. Das neue Derivat läßt sich beispielsweise herstellen indem man aus der tPA-cDNA denjenigen Abschnitt, der die dem Derivat im Vergleich zum kompletten tPA-Molekül fehlenden Aminosäuresequenz kodiert, mit den entsprechenden Restriktionsendonukleasen herausschneidet, die das gewünschte tPA-Derivat kodierenden Abschnitte verbindet, die so erhaltene tPA-Derivat-cDNA über einen geeigneten Vektor in Prokaryonten einschleust und darin exprimiert.

Das neue Derivat weist die charakteristischen biologischen Eigenschaften von tPA auf, besitzt jedoch eine verbesserte Halbwertszeit.

EP 0 242 836 A1

B e s c h r e i b u n g

Die Erfindung betrifft neue tPA-Derivate sowie Verfahren
zu deren Herstellung.

Die Hauptkomponente der Proteinmatrix von geronnenem
Blut ist polymeres Fibrin. Diese Proteinmatrix wird
durch Plasmin gelöst, das aus Plasminogen über Aktivierung durch die sogenannten Plasminogen-Aktivatoren
gebildet wird, z. B. durch tPA (Gewebs-Plasminogen-Aktivator, tissue-type plasminogen activator). Die enzymatische Aktivität von natürlichem oder aus Eukaryonten
gentechnologisch gewonnenem tPA (katalytische Aktivierung
von Plasminogen zu Plasmin) ist in Abwesenheit von
Fibrin oder Fibrinspaltprodukten (FSP) sehr gering,
kann aber in Gegenwart dieser Stimulatoren wesentlich
gesteigert werden (um mehr als den Faktor 10).

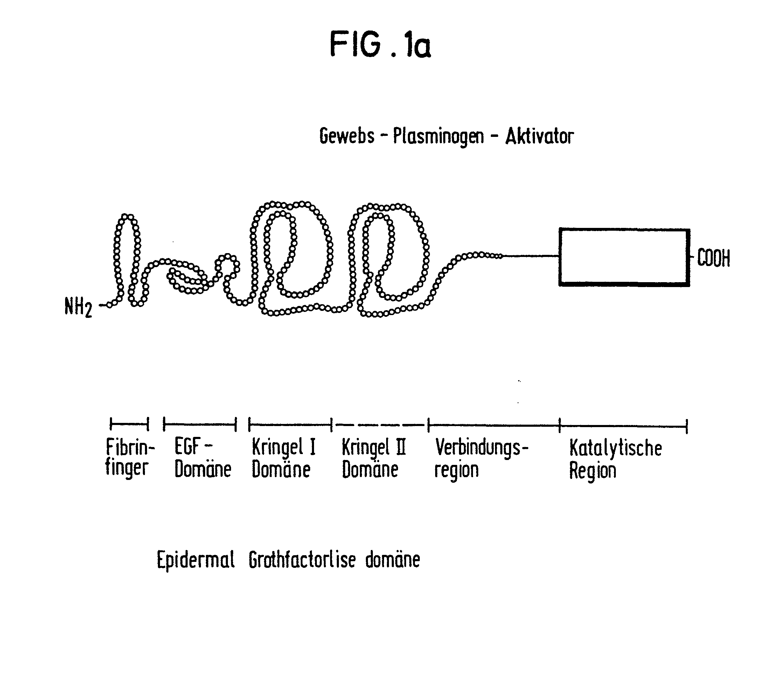

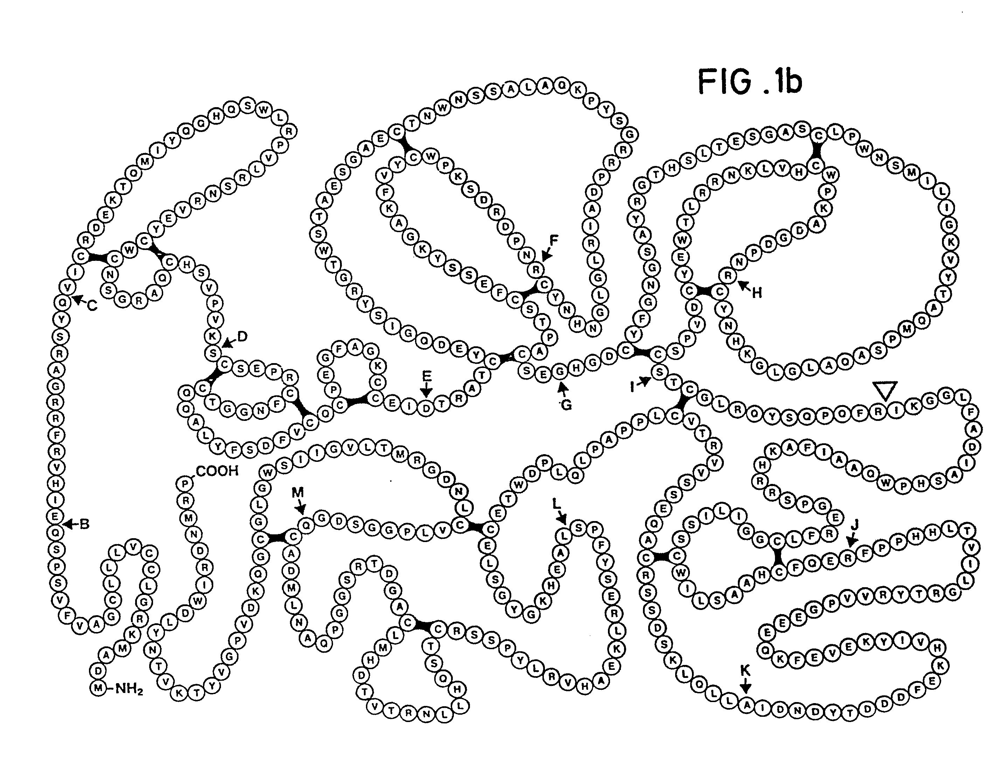

Der Wirkmechanismus von tPA in vivo ist beispielsweise
in Korniger und Collen, Thromb. Hämostasis 46, 561-565
(1981) beschrieben. Danach wird tPA durch im Blut
vorhandene Proteasen an der in Figur 1 b durch großen
Pfeil markierten Stelle in die A- und B-Kette gespalten
und aktiviert. Dabei bleiben die beiden Teilketten über
eine Cystein-Brücke verbunden. Die Stimulierbarkeit der
Aktivität ist ein entscheidender Vorteil von tPA gegenüber anderen bekannten Plasminogen-Aktivatoren, wie z.
B. Urokinase oder Streptokinase (vgl. z. B. M. Hoylaerts
et al, J. Biol. Chem. 257 (1982), 2912-2919; W. Nieuwenhuizen et al, biochemica et Biophysica Acta 755 (1983),
531-533).

Ein wesentlicher Nachteil von tPA ist jedoch seine
geringe Halbwertszeit. Aus D. Collen et al., Cicurlation 72 (1985), 384 - 388 ist bekannt, daß die Halbwertszeit von tPA bei ca. 2 Minuten liegt. Dies gilt
sowohl für den ungespaltenen Aktivator als auch für die
A-Kette allein (D.C. Rijken et al., Haemostasis 1985,
Abstract 0358). Es wird vermutet, daß tPA in der Leber
rasch aufgenommen und abgebaut wird (H. E. Fuchs et
al., Blood 65 (1985), 539-544). Durch diese geringe
Halbwertszeit sind für eine therapeutische Anwendung
von tPA sehr hohe Dosen erforderlich, was zu hohen
Behandlungskosten führt. Außerdem besteht die Befürchtung, daß tPA in derart hohen Dosen als Immunogen
wirken könnte.

Der Erfindung liegt daher die Aufgabe zu Grunde tPA-Derivate zu schaffen, welche gegenüber dem natürlichen
tPA eine wesentlich verlängerte Halbwertszeit aufweisen
und die charakteristischen biologischen tPA-Eigenschaften, nämlich die proteolytische Aktivität und die
Stimulierbarkeit der Aktivität durch Fibrin immer noch
aufweisen.

Gelöst wird diese Aufgabe erfindungsgemäß durch ein
tPA-Derivat, welches dadurch gekennzeichnet ist, daß es
eine Aminosäuresequenz aufweist, die der des natürlichen
tPA entspricht, in der jedoch die die EGF-Domäne bildenden Aminosäuren des natürlichen tPA-Moleküls mindestens
teilweise fehlen und mindestens einer der Kringel I
oder II, die Verbindungsregion, die katalytische Region
und die Fibrinfinger vorhanden sind. Im erfindungsgemäßen tPA-Derivat ist daher die Aminosäurekette des
natürlichen tPA vom $NH_2$-Ende bis einschließlich des
Fibrinfingers direkt oder über Spacer mit der Aminosäurekette des tPA hinter der EGF-Domäne insbesondere
der Kringel-II-Domäne bis zum COOH-Ende verbunden.
Außer der EGF-Domäne kann auch einer der Kringel I oder
II ganz oder teilweise fehlen.

- 3 -

Im biologisch aktiven tPA ist die Aminosäurekette in
charakteristischer Weise gefaltet und durch Disulfidbrükken intramolekular verbunden unter Bildung von charakteristischen Domänen, die vom $NH_2$-Ende her beginnend als
Fibrinfinger-Domäne (Aminosäuren 1 bis 49), EGF-Domäne
(epidermal growth factor like-Domäne) (Aminosäuren 50
bis 87), Kringeldomäne I (Aminosäuren 88 bis 176),
Kringeldomäne II (Aminosäuren 177 bis 262), Verbindungsregion und katalytische Region bezeichnet werden, wie
in Figur 1 a dargestellt. Das erfindungsgemäße Derivat
ist nunmehr dadurch gekennzeichnet, daß die sogenannte
EGF-Domäne zumindest teilweise fehlt und außerdem die
Kringeldomäne I oder II, ganz oder teilweise ebenfalls
fehlen kann. Vorzugsweise fehlt der überwiegende oder
vollständige Rest der Aminosäuren der Kringeldomäne I
bis zur 1. Disulfidbrücke der Kringeldomäne II. Die
Fibrinfingerdomäne, welche die Aminosäuren 1 bis 49 des
tPA-Moleküls umfasst, kann gekürzt sein, derart daß die
Aminosäuren 44 bis 49 ganz oder teilweise fehlen. Figur
1 b zeigt das vollständige tPA unter Angabe der Aminosäuresequenz und der Stellen, wo in der codierenden DNA
Introns herausgeschnitten werden (Pfeile mit Buchstaben),
wobei am $NH_2$-Ende eine aus 35 Aminosäuren bestehende
Leadersequenz dargestellt ist, welche bei tPA-Bildung
in Eukaryonten in dem zuerst entstehenden tPA-Vorläufer
vorliegt.

Ausgedrückt unter Bezugnahme auf die Nummerierung der
Aminosäuren in der vollständigen tPA-Sequenz fehlen
beim erfindungsgemäßen tPA-Derivat diejenigen Aminosäuren, die einen Abschnitt bilden, der mit einer der
Aminosäuren 44 bis 72 beginnt und mit einer der Aminosäuren 87 bis 179 endet. Hierbei wird die in Nature, 301
(1983) 214-221 vom Pennica aufgestellte Nomenklatur
verwendet. Vorzugsweise endet der entfernte Abschnitt
mit einer der Aminosäuren 113 (hier liegt die 1. Disulfidbrücke des Kringels I) bis 179. Falls Kringel II teilweise oder ganz fehlt sind im erfindungsgemäßen Derivat im Vergleich zu tPA zwei Abschnitte der Aminosäurekette entfernt, nämlich EGF-Domäne und der Kringel II-Teil während der dazwischenliegende Kringel I vorhanden ist.

Das erfindungsgemäße tPA-Derivat besitzt überraschenderweise eine wesentlich höhere Halbwertszeit im Organismus als das natürliche tPA und besitzt gleichzeitig die erwähnten biologischen Eigenschaften, insbesondere dessen enzymatische Aktivität und Stimulierbarkeit des tPA in unvermindertem Ausmaß. Damit wird es möglich, die durch tPA hervorgerufenen therapeutischen Effekte mit wesentlich geringeren Mengen an erfindungsgemäßen tPA-Derivat zu erzielen. Besonders gute Eigenschaften ergeben sich dabei bei tPA-Derivaten, denen die Aminosäuren 45 bis 179 bzw. 50 bis 87, bzw. 50 bis 175 des kompletten tPA-Moleküls fehlen (Molekulargewicht von etwa 43000 D).

Die Herstellung der neuen tPA-Derivate kann dadurch erfolgen, daß auf cDNA-Ebene mit geeigneten Restriktionsendonukleasen geschnitten und anschließend wieder ligiert wird. Diese modifizierte cDNA wird dann über einen geeigneten Vektor in Prokaryonten oder Eukaryonten eingeschleust und exprimiert. Bei dieser Ausführungsform des Verfahrens der Erfindung verfährt man so, daß man aus der tPA-cDNA denjenigen Abschnitt, der die dem Derivat im Vergleich zum kompletten tPA-Molekül fehlende Aminosäuresequenz kodierte, mit den entsprechenden Restriktionsendonukleasen herausschneidet, die das gewünschte tPA-Derivat kodierenden Abschnitte verbindet, die so erhaltene tPA-Derivat-cDNA über einen geeigneten Vektor in Prokaryonten einschleust und darin exprimiert.

Als Restriktionsendonukleasen sind insbesondere solche Enzyme geeignet, welche nur in dem Bereich von bp 315 bis bp 726 der cDNA-Sequenz (vgl. Pennica, Nature 301 (1983), 214 - 221) schneiden.

Besonders geeignet sind die Restriktionsendonukleasen-Kombinationen Dra III und Mae III zur Entfernung der Aminosäuren 44 bis 179 oder Dde I und Mnl I zur Entfernung der Aminosäuren 44 bis 171 sowie Dde I für die Aminosäuren 44 bis 174 und Fnu 4 H zur Entfernung der Aminosäuren 52 - 168 und Rsa I zur Entfernung von den Aminosäuren 67 - 162. Sofern Restriktionsendonukleasen verwendet werden müssen, welche die cDNA auch außerhalb des erwähnten Bereichs schneiden, kann über die Einwirkzeit eine Steuerung erreicht werden. Die entstehenden Enden der DNA-Fragmente müssen durch geeignete Auffüllung oder durch Verdau mit S1-Nuklease oder Einfügung von Linkern so modifiziert werden, daß eine phasengerechte Verknüpfung der verbleibenden Aminosäuren-Tripletts möglich wird.

Zur Behandlung mit Restriktionsendonukleasen und anschließender Ligierung ist die cDNA bevorzugt in einen Vektor eingebaut. Geeignete Vektoren sind beispielsweise pBR 322, pKK 223-3, pACYC 177, pRSF 1010, eukaryontische Vektoren, wie Bovine papilloma virus oder Shuttle-Vektoren, wie pKCR. Zur Wiederverknüpfung im Vektor werden die bekannten Methoden, vorzugsweise unter Verwendung von Linkern, angewendet.

Anschließend werden diese Vektoren in Prokaryonten und Eukaryonten nach den bekannten Verfahren eingeführt und die tPA-Derivate exprimiert. Dabei wird vorzugsweise bei Verwendung von Prokaryonten eine Steuerung der Expression in an sich bekannter Weise vorgesehen, z.B. durch Verwendung des Systems lac-Promotor (oder Derivat davon wie tac-Promotor) und lac-Repressor, oder trp-Promotor und trp-Repressor oder lambda-Promotor und lambda-Repressor.

Ebenso kann zur Herstellung der Derivate von der vollständigen DNA, welche Introns enthält, ausgegangen werden. Hierzu wird die tPA-DNA aus der Maniatis-Genbank isoliert, in Vektoren eingesetzt. Dies erfolgt über gezielte Deletion der für die im erfindungsgemäßen tPA-Derivat im Vergleich zu tPA fehlenden Aminosäuren codierenden Tripletts und der dazwischen positionierten Introns mittels Oligonukleotiden. Bei dieser Ausführungsform des Verfahrens der Erfindung verfährt man so, daß man tPA-DNA in einen geeigneten Vektor einführt, den erhaltenen Vektor dann mit einem Oligonukleotid hybridisiert, welches mit den Basensequenzen, die zu beiden Seiten an die aus dem tPA-DNA Molekül zu entfernenden Basensequenzen anschließen, komplementäre Baseseqenz aufweist, einer Reparatur-Mutagenese unterwirft, den so mutierten Vektor dann in eine geeignete Zelle einführt und amplifiziert, diejenigen amplifizierten Vektoren gewinnt, welche mit dem Oligonukleotid hybridisieren und in dafür geeignete Prokaryonten einführt und exprimiert. Das Oligonukleotid sollte vorzugsweise aus 18 bis 26 Nukleotiden bestehen. Von diesen Nukleotiden sollten vorteilhaft je die Hälfte mit den zu erhaltenen Nukleotiden der davor und dahinter liegenden Tripletts übereinstimmen. Dieses Verfahrensprinzip der "gapped-duplex"-Synthese ist in Morinaga et al Biotechnology 2 (1984), 634-639 und Nagai et al, Nature 309 (1984) 810 ausführlich beschrieben.

Desweiteren kann aus tPA-produzierenden Zellinien (Literatur Pennica, loc.cit.) die m-RNA isoliert werden und durch Größenfraktionierung aufgetrennt werden. Durch natürliche Splice-Artefakte können die m-RNA-Derivate entstehen, die verkürzt sind. Durch c-DNA-Herstellung daraus und Untersuchung der entstandenen Klone können die Klone gefunden werden, welche die codierende Region für die Aminosäuren, die dem tPA-Derivat im Vergleich zu tPA fehlen, insbesondere die Aminosäuren 49 bis 175, nicht mehr enthalten. Dazu werden Klone ausgewählt, die mit entsprechend konstruierten Oligonukleotiden, passend für die entfernte Region, nicht mehr hybridisieren. Solche verkürzten cDNA-Moleküle können auch durch Fehler bei der cDNA-Herstellung aus intakter oder partiell deletierter mRNA prinzipiell entstehen und durch Hybridisierung und Sequenzierung aufgefunden werden.

Ebenso kann tPA-Derivatisierung auf Aminosäureebene erfolgen. tPA, beispielsweise gentechnologisch hergestellt und gereinigt, wird dann mit Proteasen, welche im Bereich zwischen den Aminosäuren 43 und 72 und zwischen 87 und 179 schneiden, geschnitten und wieder zusammengefügt. Die zu vereinigenden Fragmente lassen sich durch Gelelektrophorese oder andere Größenfraktionierung von anderen Fragmenten isolieren.

Bei den erfindungsgemäßen tPA-Derivaten kann es vorteilhaft sein, den Abstand Fibrinfinger - Kringel II - zu optimieren, um die tPA-charakteristische enzymatische Wirksamkeit des Moleküls maximal zur Wirkung zu bringen. Dies kann z.B. durch Einbau kurzer Aminosäureketten oder anderer Distanzglieder (Spacer) zwischen Fibrinfinger-Domäne und Kringel-II-Domäne erfolgen.

Besonders bevorzugt ist ein tPA-Derivat, bei dem die Aminosäuren 45 bis 179 fehlen. Dieses kann beispielsweise auf cDNA-Ebene hergestellt werden, durch Schnitt mit den Restriktionsendonukleasen Dra III und Mae III und Verdau der überstehenden Einzelstrangenden mit Nuclease

S1. Anschließend wird mit Ligase ligiert. Dann wird mit einem geeigneten Vektorsystem, wie es z.B. in der deutschen Patentanmeldung mit dem Aktenzeichen P 36 13 401.5 beschrieben ist, in Prokaryonten oder Eukaryonten exprimiert. Das bei der Expression in Pro- oder Eukaryonten entstandene tPA-Derivat hat ein Molekulargewicht von ca 43000 D. Das in Eukaryonten entstandene Derivat ist zusätzlich glycolisiert und wandert daher in der SDS-Elektrophorese etwas verlangsamt.

Ein weiteres, besonders bevorzugtes tPA-Derivat unterscheidet sich vom tPA durch Fehlen der Aminosäuren 50 bis 175. Zur Herstellung eignet sich die oben beschriebene tPA-DNA Methode der "gapped-duplex"-Synthese besonders, weil sie nicht das Vorhandensein von Restriktionsschnittstellen an den gewünschten Positionen der Sequenz voraussetzt.

Die Erfindung schafft neue pharmakologisch wirksame Proteine die sich vom tPA ableiten, aber eine verbesserte Kombination von Eigenschaften aufweisen, verglichen mit tPA, sowie Verfahren zu ihrer Herstellung.

B e i s p i e l   1

Deletion der die Halbwertzeit bestimmenden Domänen von tPA

1a)   Konstruktion des tPA-Mutein-Gens

Als Ausgangsplasmid dient das Plasmid pBT 95 (DSM 3611 P, DE 35 45 126), aus dem das Plasmid pePa 98.1 auf folgende Weise hergestellt wurde: Das Plasmid wird mit dem Enzym Bgl II gespalten und mit der Nuclease S1 nachbehandelt. Durch weitere

Spaltung mit dem Enzym Sca I läßt sich ein Fragment a präparativ erhalten, das ca. 760 Basenpaare groß ist und die für tPA kodierende Nucleotid-Sequenz 192-952 (Pennica loc.cit.) enthält. Ein Fragment b wird ebenfalls aus pBT 95 erhalten durch Spaltung mit Sca I und Hind III; dadurch erhält man ein Fragment von etwa 1200 Basenpaaren, das die tPA-Nucleotid-Sequenz 953-2165 enthält. Ein Partner c wird als Linker synthetisch hergestellt und enthält die folgende Sequenz:

5'GAATTCTTATGTC3'
3'       GAATACAG5'

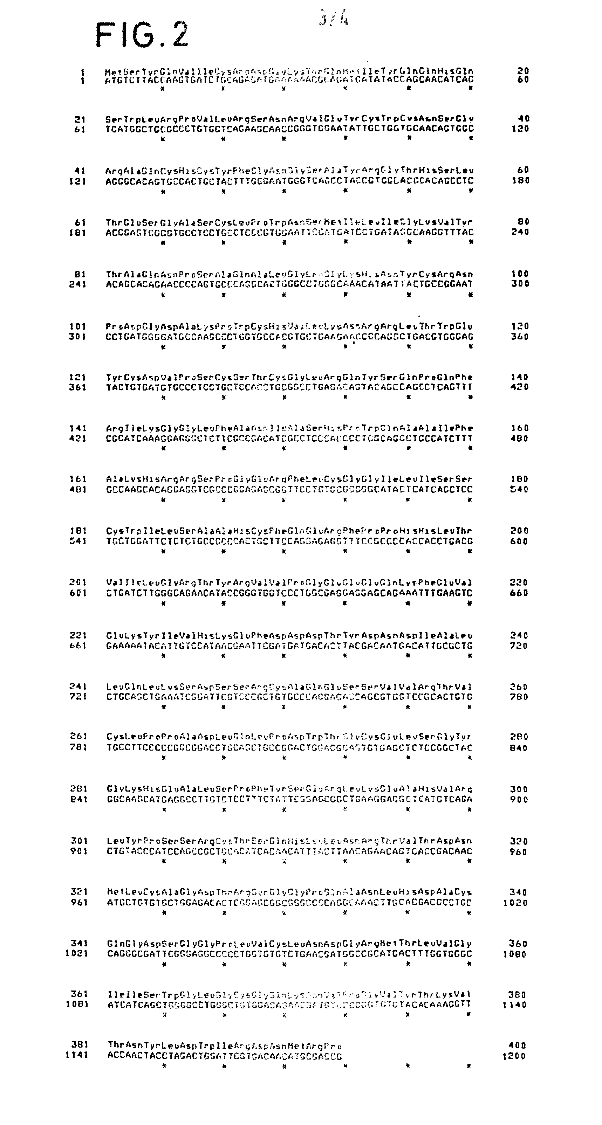

Als Partner d in der Konstruktion wird das Plasmid pKK 233-3 (DSM 3694 P) mit den Enzymen Eco RI und Hind III im Polylinker gespalten. Die Partner a-d werden zusammen ligiert. Der Ligationsansatz wird nach üblicher Technik mit T4-Ligase behandelt und anschließend in E.coli-Zellen (DSM 3689) transformiert. Die transformierten Zellen werden auf Medium unter Zusatz von 50µg/ml Ampicillin kultiviert. Aus den erhaltenen Klonen werden diejenigen ausgewählt, die das Plasmid pePa 98.1 tragen, das sich von den Ausgangsplasmiden pBT 95 und pKK 223-3 dadurch unterscheidet, daß es im Polylinker des Plasmides pKK 223-3 zwischen der Eco RI und Hind III-Spaltstelle die tPA-Nucleotid-Sequenz 192-2165 in richtiger Reihenfolge enthält. Aus diesem Plasmid pePa 98.1 wurde das tPA-codierende Fragment mit dem tac-Promotor über die Xho II-Spaltung erhalten. Dieses Fragment ist etwa 1,85 kb groß und wird an den überstehenden Enden durch Behandlung mit Klenow-Enzym, in Gegenwart von allen vier Desoxynukleosidtriphosphaten vollständig aufgefüllt. In einem Schritt A wird dieses Fragment mit dem Enzym Dra III geschnitten und die
überstehenden Enden mit der Nuclease S1 abgedaut.
Gelelektrophoretisch wird das Fragment der Größe
von ca. 370 Basenpaaren gewonnen. In einem Schritt
B wird das gleiche Xho II Ausgangsfragment mit dem
Enzym Mae III geschnitten und ebenfalls mit S1
nachverdaut. Anschließend wird dieser Ansatz
zusätzlich mit dem Enzym Sac I behandelt. Gelelektrophoretisch wird daraus ein Fragment der Größe von
ca. 700 Basenpaaren isoliert. In einem Ansatz C
wird das Vektorplasmid pePa 98.1 mit dem Enzym
BamH I geschnitten und die überstehenden Enden mit
dem Klenow-Enzym, unter Einsatz von allen vier
Desoxynukleosidtriphosphaten aufgefüllt und mit
dem Enzym Sac I nachgespalten. Gelelektrophoretisch
wird anschließend das größte Fragment aus diesem
Ansatz gewonnen. In einem Ligierungsansatz werden
die gewonnenen Fragmente aus den Ansätzen A, B und
C vereinigt und unter Zusatz von DNA-Ligase ligiert.
Die Ligierungsmischung wird in E.coli-Zellen (DSM
3689), welche ein lac I$^q$-Plasmid enthalten, transformiert und die entstehenden Transformanten auf
Nährmedium mit 50 µg/ml Ampicillin selektioniert.
Aus den so erhaltenen Klonen werden diejenigen
ausgewählt, die das gewünschte Plasmid pePa 129
enthalten, das sich vom Ausgangsplasmid pePa 98.1
dadurch unterscheidet, daß es die DNA-Sequenz
zwischen der tPA cDNA-Sequenz Pos. 301 bis 726
nicht mehr enthält. Figur 2 zeigt die Nucleotidsequenz des so erhaltenen Muteins und die daraus
erhaltene Aminosäuresequenz, in der die Aminosäuren
45 bis 179 des tPA fehlen.


1b)   Konstruktion eines Expressionsvektors für das
      Mutein zur Expression in E.coli

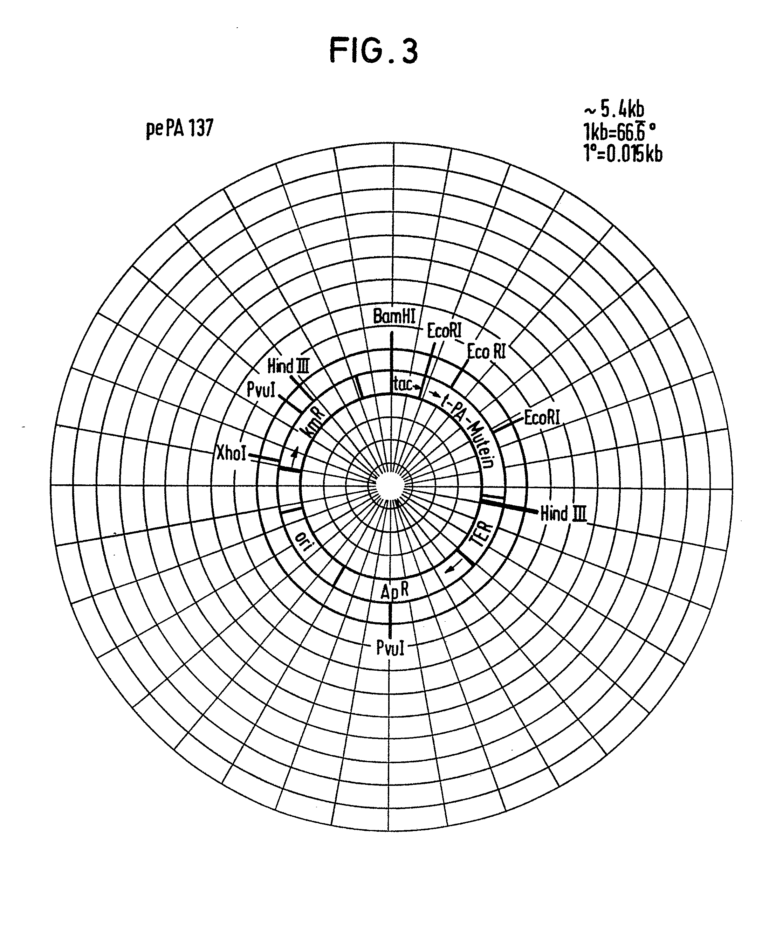

Aus dem nach 1a) hergestellten Plasmid pePa 129
wird durch Verdau mit den Restriktionsenzymen Bam
H I und Pvu I das tPA-codierende Fragment erhalten.
Das Plasmid pACYC 177 (DSM 3693 P) wird ebenfalls
mit Bam H I und limitiert mit Pvu I gespalten.
Beide Fragmente werden zusammenligiert und in
E.coli-Zellen (DSM 3689), welche ein lac I$^q$-Plasmid
enthalten, transformiert. Durch Selektion auf
Kanamycin und Ampicillin mit je 25 bzw. 50 µg/ml
erhält man Transformanten, unter denen diejenigen
ausgewählt werden, die das Plasmid pePa 137 enthalten. Dieses unterscheidet sich von den Ausgangsvektoren dadurch, daß es das Mutein- Genfragment aus
pePa 129 und die Sequenz des Plasmides pACYC 177
jeweils enthält. Eine Restriktionsschnittstellenkarte
des Plasmides pePa 137 zeigt Figur 3.

1c)  Konstruktion eines Expressionsvektors für das
     Mutein zur Expression in Pseudomonas putida

Als Vektor für Pseudomonas putida wird ein Derivat
des Plasmides pRSF 1010 verwendet, welches zusätzlich ein Kanamycin-Resistenzgen aus pACYC 177
enthält. Dieser Vektor führt die Bezeichnung pREM
3061 (DSM 3692 P). Dieses Plasmid wird durch
Verwendung des Restriktionsenzyms Hpa I linearisiert.
Aus dem nach 1 a) hergestellten Vektor pePa 129
wird durch Xho II-Spaltung ein ca. 1,45 kb großes
Fragment gewonnen, das den tac-Promotor und die
vollständige Mutein- Gensequenz enthält. Durch
Behandlung mit Klenow-Fragment, in Gegenwart von
allen vier Desoxynukleosidtriphosphaten werden die
Xho II-Schnittstellen vollständig aufgefüllt. Das
so behandelte Fragment wird mit dem linearisierten
Vektor pREM 3061 ligiert und in E.coli-Zellen (DSM
3698), transformiert. Die Transformanten werden auf Medium mit 25 µg/ml Kanamycin selektioniert.
Transformanten, die das Plasmid pePa 143 enthalten,
werden ausgewählt. Dieses Plasmid unterscheidet
sich vom Ausgangsvektor pREM 3061 dadurch, daß es
die vollständige Mutein- Gensequenz mit tac-Promotor
enthält. Dieses Plasmid wird isoliert und durch
Transformation in Zellen des Stammes Pseudomonas
putida, DSM 2106, eingeführt. Die Transformanten
werden auf Medium mit 25 µg/ml Kanamycin selektioniert und anschließend analysiert. Sie enthalten
das Plasmid pePa 143. Zusätzlich kann in diese
Transformanten-Zellen ebenfalls durch Transformation ein Plasmid pePa 119 (DSM 3691 P) eingeführt
werden, das mit pePa 143 kompatibel ist und das
lac I$^q$-Gen enthält. Dieses Plasmid ist ein Derivat
vom Plasmid RP 4. Die Anwesenheit dieses Plasmides
unterdrückt die Produktion des Muteins in den
Zellen durch Produktion des lac-Repressor-Proteins.
Dieses kann die Transkription vom tac-Promotor aus
reprimieren.


B e i s p i e l   2


Konstruktion von Vektoren, die das tPA-Muteingen mit
Leader-Sequenz zur Sekretion enthalten und für die
Expression in CHO-Zellen geeignet sind.


Verwendet wird der Vektor pKCR. Ein Plasmid, das die
vollständige tPA cDNA enthält, ist das Plasmid pBT 95.
Dieses Plasmid wird mit dem Restriktionsenzym Pst I
limitiert gespalten, so daß ein Fragment gewonnen wird,
das die tPA-Sequenz von der cDNA, Nucleotidposition 205
- 1312, nicht mehr enthält. Aus dem nach 1a) hergestellten Plasmid pePa 129, das das Muteingen enthält, wird
durch limitierte Pst I-Spaltung das Fragment gewonnen, das die Nucleotidposition 205 - 1312 enthält, aber eine
Deletion ausweist für die Sequenz von 322 - 726. Diese
so gewonnenen Fragmente werden gemeinsam ligiert und in
E.coli-Zellen (DSM 3689), transformiert. Unter Zusatz
von 50 μg/ml Ampicillin werden Transformanten gewonnen
und die Klone ausgewählt, die das Muteinfragment in
richtiger Orientierung inseriert bekommen haben. Diese
Plasmide werden mit pePa 144 bezeichnet. Sie werden in
CHO-Zellen, die DHFR⁻ sind, nach der Methode von R.
Kaufmann und P. Sharp I. Mol. Biol. $\underline{15}$, 601-621 (1982)
eingeführt. Unter den dort beschriebenen Bedingungen
wird tPA-Mutein exprimiert und analysiert (Molec. Cell.
Biol. 5, 1750-1759 (1985).

B e i s p i e l    3

Expression des tPA-Muteins in E.coli-Zellen

Das nach 1a) und 1b) hergestellte Plasmid pePa 137 wird
in E.coli-DSM 3698 transformiert, der ein lac I$^q$-Plasmid
enthält. Die Transformanten werden auf Medium mit 50 μg
Ampicillin selektioniert. Die Zellen, die dieses Plasmid
tragen, werden in Nährboulillon bis zu OD$_{550nm}$ = 0,4
unter Belüftung gezüchtet und dann unter Zusatz von 0,2
% Lactose oder 10 mmol/l IPTG induziert. Die Bebrütung
geschieht jeweils bei 37°C. Nach 4 Stunden Bebrütung
unter Belüftung werden die Zellen geerntet und aufgeschlossen. Dazu werden sie für 15 Minuten auf Eis mit
0,5 mg/ml Lysozym behandelt (Zellkonzentration 10
OD/ml). Verwendet wird Tris-Puffer, pH 8,6 (0,1 mol/l
Tris HCL mit 10 mmol/l EDTA und 100 mmol/l NaCl).
Danach werden die Zellen durch Ultraschallbehandlung
aufgeschlossen. Die so erhaltene Suspension wird für 10
Minuten bei 20 000 g zentrifugiert und der Überstand
verworfen. Das Sediment enthält das tPA-Mutein in
inaktiver Form. Dieses kann nach der in DE 35 37 708 beschriebenen Methode gelöst und reaktiviert werden.
Das gewonnene Pellet kann auch durch Zusatz von 1 % SDS
und 100 mmol/l Mercaptoethanol gelöst werden und in
einem SDS Polyacrylamid-Gel elektrophoretisch aufgetrennt werden. Durch Anfärbung der Proteine nach erfolgter Elektrophorese mittels Coomassie-blue können
die Proteinbanden sichtbar gemacht werden. Der Extrakt
enthält gem. dieser Analyse hauptsächlich ein Protein
des Molekulargewichts von ca. 43 000 Dalton. Das tPA-
Mutein ist, wie erwartet, kleiner als das aus analog
behandelten Zellen mit pePa 133 gewonnene intakte tPA
(Molekulargewicht ca 57 000 Dalton). Sowohl tPA-Mutein
als auch tPA können nach Westernblot-Verfahren mit
Anti-tPA PAK-Konjugat aus Ziege als immunologisch
gleichwertig nachgewiesen werden.


B e i s p i e l     4

Vergleich von Mutein und t-PA bezüglich der proteolytischen Aktivität und der Stimulierbarkeit durch Fibrin.

Gem. Beispiel 2 wird aus Bakterien, die das Plasmid
pePa 137 oder pePa 133 enthalten, das Mutein oder tPA
gewonnen. Nach dem in DE 35 37 708 beschriebenen Verfahren wird entsprechend aktives Mutein oder tPA erhalten.
Alle Proteine liegen in einkettiger Form in Lösung vor.
Dabei erhält man vergleichbare Ausbeuten bezogen auf
die Proteinausgangskonzentration (Tabelle 1). Im Test
auf proteolytische Aktivität werden für beide Präparationen vergleichbare Werte erhalten (Tabelle 1). Die
proteolytische Aktivität wird hierbei wie in DE 3537708
bestimmt. Der Vergleich der Aktivität, die man mit und
ohne Zusatz von Fibrin-Fragmenten erhält, zeigt vergleichbare Werte für beide Präparationen (Tabelle 1).
Demnach ist das Mutein so von tPA nicht zu unterscheiden, sondern identisch in seinen enzymatischen Eigenschaften.

<u>Tabelle 1:</u>

|  | tPA | Mutein |
|---|---|---|
| mg-Proteinausbeute | 234,0 | 75,0 |
| Davon mg-mit Aktivität | 11,6 | 5,6 |
| Faktor der Stimulierbarkeit | 11,0 | 21,6 |

B e i s p i e l  5

Bestimmung der Halbwertszeit.

Das nach Beispiel 1 - 3 hergestellte Mutein hat eine höhere Halbwertszeit im biologischen Test. Präparationen von tPA und Mutein, die nach Beispiel 3 erhalten wurden, wurden dazu im Maus-Test-System verglichen. Verwendet wurde das von Fuchs et al beschriebene Verfahren (Blood 65 (1985), 539-544).

Gefunden wurden vergleichbare Werte für die Plasmaclearence für tPA. Das Mutein hingegen wurde langsamer aus dem Blut entfernt, nachgewiesen durch TCA-präzipitierbares $^{125}$I-Mutein. Die Akkumulation in der Leber war deutlich herabgesetzt, erst nach 20 bis 30 min. wurden Werte gemessen, die für tPA schon nach 5 min. erreicht worden waren.

B e i s p i e l    6

Deletion der die Halbwertszeit bestimmenden Domänen von
t-PA mittels Oligo-Nukleotid induzierter Mutagenese.

6a)    Herstellung des t-PA-Vektors pePa 133

Aus dem gemäß Beispiel 1a) hergestellten Plasmid
pePa 98.1 wird durch Spaltung mit dem Enzym Xho II
ein ca. 1,85 kb großes Fragment erhalten und präparativ isoliert. Dieses Fragment enthält die tac-
Promoter- und lac-Operator-Region mit einem ATG
Startcodon und der t-PA-Nukleotid-Sequenz 192-1809
in richtiger Reihenfolge. Das Plasmid pKK 223-3
(DSM 3694 P) wird mit dem Enzym Bam HI gespalten,
das größere Fragment präparativ gewonnen und mit
dem Xho II-Fragment aus pePa 98.1 vereinigt und
unter Zusatz von DNA-Ligase ligiert. Die Ligierungsmischung wird in E.coli-Zellen (DSM 3689),
welche ein lac I$^q$-Plasmid enthalten, transformiert
und die entstehenden Transformanten auf Nährmedium
mit 50 µg/ml Ampicillin selektioniert. Aus den so
erhaltenen Klonen werden diejenigen ausgewählt,
die das gewünschte Plasmid pePa 126 enthalten.
Dieses unterscheidet sich vom Ausgangsplasmid pePa
98.1 dadurch, daß mit Bam HI- und Hind III-Spaltung ein ca. 1,85 kb großes Fragment neben einem
ca. 4,4 kb großen Fragment erhalten wird.

Aus diesem Plasmid pePa 126 wird durch Verdauung
mit den Enzymen Bam HI und Pvu I ein Fragment erhalten, das ca. 2,9 kb groß ist und die t-PA
codierende Sequenz enthält. Das Plasmid pACYC 177 (DSM 3693 P) wird ebenfalls mit Bam HI und limitiert mit PvuI gespalten. Das dabei entstehende
größere Fragment wird zusammen mit dem t-PA
codierenden Fragment aus pePa 126 unter Zusatz von
T4-Ligase ligiert und in E. coli-Zellen (DSM 3689),
welche ein lac I$^q$-Plasmid enthalten, transformiert.
Durch Selektion auf Nährmedium mit Kanamycin und
Ampicillin mit je 25 bzw. 50 µg/ml erhält man
Transformanten, unter denen diejenigen ausgewählt
werden, die das Plasmid pePa 133 enthalten. Dieses
unterscheidet sich von den Ausgangsvektoren dadurch,
daß es das ca. 1,85 kb große t-PA-Genfragment
(nach Spaltung mit Bam HI und Hind III) aus pePa
126, die Sequenz des Plasmids pACYC 177 enthält.

6d)  Deletion der EGF-Kringel I-Domänen aus pePa 133

Prinzipiell wird die Methode von Morinaga et al
Biotechnology 2 (1984) 634-639 verwendet. Es
werden zwei Spaltansätze von pePa 133 hergestellt.
Ansatz a wird mit Eco RI gespalten und das größte
Fragment isoliert. Ansatz b wird mit Xho I gespalten und so das Produkt linearigiert. Man vereinigt
das Fragment aus Ansatz a mit Ansatz b (je ca. 150
fmol) und denaturiert beide DNAs durch kurzes
Erhitzen auf 95°C. Anschließend wird diese Mischung
bei 60°C für ca. 30' inkubiert. Zusätzlich wird
ein Oligo-Nukleotid (75 fmol) dazugefügt. Seine
Sequenz lautet:

5'GCCTGTCAAAGGAAACAGTGA 3'

Nach Abkühlung der Mischung auf 12°C werden hinzugefügt: Desoxynucleotidtriphosphate (0,25 mM), ATP
(1 mM), NaCl (100 mM), Tris-HCl pH 7,5 (6,5 mM),
MgCl$_2$ (8mM), ß-Mercaptoäthanol (1 mM), Klenow-Fragment der DNA-Polymerase aus E. coli (0,125 U/µl Ansatz), T4-Ligase (0,0625 U/µl Ansatz). Die Mischung wird für 4 Stunden bei 12°C belassen. Anschließend wird dieser Ansatz in E. coli-Zellen (DSM 3689), welche ein lac I^q-Plasmid enthalten, transformiert. Durch Selektion auf Kanamycin und Ampicillin mit je 25 bzw. 50 µg/ml Nährmedium erhält man Transformanten. Unter diesen werden diejenigen ausgewählt, die das Plasmid pREM 7685 enthalten. Dieses unterscheidet sich vom Ausgangsvektor dadurch, daß es mit dem genannten Oligo-Nukleotid spezifisch hybridisiert (Waschtemperatur 50°C) und ein verkürztes Eco RI-Fragment enthält. Statt eines ca. 0,61 kb Fragment in pePa 133 ist nur ein 0,23 kb Fragment neben den übrigen Eco RI-Fragmenten nachweisbar.

Das so erhaltene t-PA-Muteingen kann wie in Beispiel 3 beschrieben, exprimiert und ein Protein der Größe ca. 43000 d, welches die Sequenz eines natürlichen tPA aufweist in welcher die Aminosäuren 50 bis 175 fehlen, d.h. die Aminosäuren 49 und 176 miteinander verknüpft sind, nachgewiesen werden. Wie in Beispiel 4 beschrieben, kann es ebenfalls als aktives Mutein erhalten werden. Seine Werte sind nicht unterschiedlich von den in Tabelle 1 beschrieben. Wie in Beispiel 5 beschrieben, wird im Maus-Test-System eine ebenso verlangsamte Plasmaclearence und deutlich herabgesetzte Leber-Akkumulation gefunden.

Beispiel    7

Deletion der EGF-Domäne aus pePa 133

Aus dem gemäß Beispiel 6a hergestellten t-PA-Vektor
pePa 133 werden gemäß Beispiel 6b) die Spaltansätze a)
und b) hergestellt und wie dort behandelt. Zusätzlich
wird dem dort beschriebenen Gemisch jedoch folgendes
Oligonukleotid (75 μmol) zugefügt:

5' GCCTGTCAAAACCAGGGCC  3'

Alle weiteren Schritte werden wie in Beispiel 6b) beschrieben, durchgeführt. Es werden diejenigen Klone
ausgewählt, die das gesuchte Plasmid pREM 7732 enthalten. Dieses unterscheidet sich vom Ausgangsvektor durch
ein verkürztes EcoRI-Fragment, statt ca. 0,61 kb ist
dieses nur noch ca. 0,46 kb groß.

Das so erhaltene Muteingen kann wie in Beispiel 3
beschrieben exprimiert und als Protein der Größe ca.
49.000 d mit der Sequenz eines natürlichen tPA-Moleküls
in welcher die Aminosäuren 50 bis 87 fehlen, d.h. die
Aminosäuren 49 und 88 miteinander verknüpft sind nachgewiesen werden. Wie in Beispiel 4 beschrieben, kann es
als ebenfalls aktives Mutein erhalten werden.

## Patentansprüche

1. Gewebs-Plasminogen-Aktivator (tPA) - Derivat, dadurch gekennzeichnet, daß es eine Aminosäuresequenz aufweist, die der des natürlichen tPA entspricht, in der jedoch die die EGF-Domäne bildenden Aminosäuren des natürlichen tPA-Moleküls mindestens teilweise fehlen und mindestens einer der Kringel I oder II, die Verbindungsregion, die katalytische Region und der Fibrinfinger vorhanden sind.

2. tPA-Derivat nach Anspruch 1, dadurch gekennzeichnet, daß die an die EGF-Domäne anschließenden Aminosäuren bis mindestens zur ersten Disulfidbrücke der Domäne des Kringels I fehlen.

3. tPA-Derivat nach Anspruch 2, dadurch gekennzeichnet, daß die gesamten Aminosäuren der Domäne des Kringels I bis zur ersten Disulfidbrücke der Domäne des Kringels II fehlen.

4. tPA-Derivat nach einem der vorhergehenden Ansprüche, dadurch gekennzeichnet, daß vom vollständigen tPA-Molekül ein Sequenzabschnitt fehlt, der mit einer der Aminosäuren 44 bis 72 beginnt und mit einer der Aminosäuren 87 bis 179 endet.

5. tPA-Derivat nach Anspruch 4, dadurch gekennzeichnet, daß der fehlende Sequenzabschnitt mit einer der Aminosäuren 113 bis 179 endet.

6. tPA-Derivat nach Anspruch 4, d a d u r c h g e k e n n z e i c h n e t , daß es die Aminosäuresequenz des tPA ohne die Aminosäuren 45 bis 179 aufweist.

7. tPA-Derivat nach Anspruch 4, d a d u r c h g e k e n n z e i c h n e t , daß es die Aminosäuresequenz des tPA ohne die Aminosäuren 50 bis 175 aufweist.

8. tPA-Derivat nach Anspruch 4, d a d u r c h g e k e n n z e i c h n e t, daß es die Aminosäuresequenz des tPA ohne die Aminosäuren 50 bis 87 aufweist.

9. Verfahren zur Herstellung eines tPA-Derivats nach einem der Ansprüche 1 bis 8, d a d u r c h g e k e n n z e i c h n e t , daß man aus der tPA-cDNA denjenigen Abschnitt, der die dem Derivat im Vergleich zum kompletten tPA-Molekül fehlende Aminosäuresequenz kodiert, mit den entsprechenden Restriktionsendonukleasen herausschneidet, die das gewünschte tPA-Derivat kodierenden Abschnitte verbindet, die so erhaltene tPA-Derivat-cDNA über einen geeigneten Vektor in Prokaryonten einschleust und darin exprimiert.

10. Verfahren nach Anspruch 9, d a d u r c h g e k e n n z e i c h n e t , daß man Restriktionsendonukleasen verwendet, die nur im Bereich der Basenpaare 315 bis 726 der tPA-cDNA-Sequenz schneiden.

11. Verfahren nach Anspruch 9 oder 10, d a - durch        gekennzeichnet, daß
man Dde I alleine oder zusammen mit Mnl I oder Dra
III und Mae III oder Fnu 4 H alleine oder Rsa I
alleine als Restriktionsendonukleasen verwendet.

12. Verfahren nach einem der Ansprüche 9 bis 11,
    dadurch        gekennzeichnet,
    daß man als Vektor pBR 322, pKK223-3, pACYC 177,
    pRSF 1010, Bovine papilloma virus oder pKCR verwendet.

13. Verfahren zur Herstellung eines tPA-Derivats nach
    einem der Ansprüche 1 bis 8,        dadurch
    gekennzeichnet,        daß man tPA-DNA
    in einen geeigneten Vektor einführt, den erhaltenen
    Vektor dann mit einem Oligonukleotid hybridisiert,
    welches mit den Basensequenzen, die zu beiden
    Seiten an die aus dem tPA-DNA Molekül zu entfernenden Basensequenzen anschließen, komplementäre
    Basensequenz aufweist, einer Reparatur-Mutagenese
    unterwirft, den so mutierten Vektor dann in eine
    geeignete Zelle einführt und amplifiziert, diejenigen amplifizierten Vektoren gewinnt, welche mit
    dem Oligonukleotid hybridisieren und in dafür
    geeignete Prokaryonten einführt und exprimiert.

14. Verfahren nach Anspruch 13,        dadurch
    gekennzeichnet,        daß man ein
    Oligonukleotid mit 18 bis 26 Nukleotiden verwendet.

15. Verfahren zur Herstellung eines tPA-Derivats nach
    einem der Ansprüche 1 bis 8,        dadurch
    gekennzeichnet,        daß man die
    tPA-mRNA von tPA produzierenden Zellen isoliert, nach Größe fraktioniert, Fraktionen der dem gewünschten tPA-Derivat entsprechenden Größe in cDNA umschreibt und kloniert, diejenigen Klone auswählt, die nicht mehr mit einem Oligonukleotid hybridisieren, welches mit derjenigen Region der tPA codierenden cDNA hybridisiert, die in der das tPA-Derivat codierenden cDNA gegenüber der tPA-cDNA fehlt, daraus die tPA-Derivat-DNA gewinnt und über einen geeigneten Vektor in Prokaryonten-Zellen einschleust und darin exprimiert.

16. Verfahren zur Herstellung eines tPA-Derivats nach einem der Ansprüche 1 bis 8, d a d u r c h g e k e n n z e i c h n e t , daß man tPA mit Proteasen zwischen den Aminosäuren 44 und 72 und zwischen 87 und 179 schneidet und die gebildeten Endteile wieder verbindet.

17. Verfahren nach Anspruch 16, d a d u r c h g e k e n n z e i c h n e t , daß man den Abstand zwischen Fibrinfinger und Kringeldomäne II optimiert.

18. Verfahren nach einem der Ansprüche 9 bis 17, d a d u r c h g e k e n n z e i c h n e t , daß man die Amplifikation und/oder die Expression in einem E.coli-Stamm durchführt, der ein lac I$^q$- Plasmid enthält.

19. Verfahren nach einem der Ansprüche 9 bis 17, d a d u r c h g e k e n n z e i c h n e t , daß man die Expression in Maus LTK⁻-Zellen durchführt.

20. Verfahren nach Anspruch 18, d a d u r c h g e k e n n z e i c h n e t , daß man E.coli DSM 3698 verwendet.

21. Verfahren nach Anspruch 20, d a d u r c h
g e k e n n z e i c h n e t , daß man E.coli
DSM 3698, welcher Plasmid pePa 137 enthält,
züchtet.

22. Verfahren nach einem der Ansprüche 9 bis 17,
d a d u r c h g e k e n n z e i c h n e t ,
daß man die Amplifikation oder/und Expression in
Pseudomonas putida DSM 2106 durchführt.

23. Verfahren nach Anspruch 22, d a d u r c h
g e k e n n z e i c h n e t , daß man Pseudomonas putida DSM 2106, welcher Plasmid pePa 143 und
gegebenenfalls pePa 119 enthält, züchtet.

24. Plasmid pePa 137

25. Plasmid pePa 143

26. Plasmid pePa 144

27. Plasmid pREM 7685

28. Plasmid pREM 7732

29. Mittel zur Auflösung von Gerinnseln enthaltend ein
Gewebs-Plasminogen-Aktivator (tPA)-Derivat, das
eine Aminosäuresequenz aufweist, die der des
natürlichen tPA entspricht, in der jedoch die
EGF-Domäne bildenden Aminosäuren und zumindest die
darin anschließenden Aminosäuren bis mindestens
zur ersten Disulfidbrücke der Domäne des Kringels
I des natürlichen tPA-Moleküls fehlen.

30. Mittel nach Anspruch 29, dadurch gekennzeichnet, daß es ein tPA-Derivat enthält, das die Aminosäuresequenz des tPA ohne die Aminosäuren 45 bis 170 aufweist.

31. Mittel nach Anspuch 29, dadurch gekennzeichnet, daß es ein tPA-Derivat enthält, daß die Aminosäuresequenz des tPA ohne die Aminosäuren 50 bis 175 aufweist.

32. Mittel nach Anspruch 29, dadurch gekennzeichnet, daß es ein tPA-Derivat enthält, daß die Aminosäuresequenz des tPA ohne die Aminosäuren 50 bis 87 aufweist.

# FIG . 1a

Gewebs – Plasminogen – Aktivator

| Fibrin-finger | EGF-Domäne | Kringel I Domäne | Kringel II Domäne | Verbindungs-region | Katalytische Region |

Epidermal Grothfactorlise domäne

# FIG.2

3/4

```
    1   MetSerTyrGlnValIleCysArgAspGlyLysThrGlnHisIleTyrGlnGlnHisGln          20
    1   ATGTCTTACCAAGTGATCTGCAGAGATGGAAAAACGCAGATCATATACCAGCAACATCAG          60
            x         x         x         x         x         x

   21   SerTrpLeuArgProValLeuArgSerAsnArgValGluTyrCysTrpCysAsnSerGly          40
   61   TCATGGCTGCGCCCTGTGCTCAGAAGCAACCGGGTGGAATATTGCTGGTGCAACAGTGGC         120
            x         x         x         x         x         x

   41   ArgAlaGlnCysHisCysTyrPheGlyAsnGlySerAlaTyrArgGlyThrHisSerLeu          60
  121   AGGGCACAGTGCCACTGCTACTTTGGGAATGGGTCAGCCTACCGTGGCACGCACAGCCTC         180
            x         x         x         x         x         x

   61   ThrGluSerGlyAlaSerCysLeuProTrpAsnSerMetIleLeuIleGlyLysValTyr          80
  181   ACCGAGTCGGGTGCCTCCTGCCTCCCGTGGAATTCCATGATCCTGATAGGCAAGGTTTAC         240
            x         x         x         x         x         x

   81   ThrAlaGlnAsnProSerAlaGlnAlaLeuGlyLeuGlyLysHisAsnTyrCysArgAsn         100
  241   ACAGCACAGAACCCCAGTGCCCAGGCACTGGGCCTGGGCAAACATAATTACTGCCGGAAT         300
            x         x         x         x         x         x

  101   ProAspGlyAspAlaLysProTrpCysHisValLeuLysAsnArgArgLeuThrTrpGlu         120
  301   CCTGATGGGGATGCCAAGCCCTGGTGCCACGTGCTGAAGAACCCCAGCCTGACGTGGGAG         360
            x         x         x         x         x         x

  121   TyrCysAspValProSerCysSerThrCysGlyLeuArgGlnTyrSerGlnProGlnPhe         140
  361   TACTGTGATGTGCCCTCCTGCTCCACCTGCGGCCTGAGACAGTACAGCCAGCCTCAGTTT         420
            x         x         x         x         x         x

  141   ArgIleLysGlyGlyLeuPheAlaAsnIleAlaSerHisProTrpGlnAlaAlaIlePhe         160
  421   CGCATCAAAGGAGGGCTCTTCGCCGACATCGCCTCCCACCCCTGGCAGGCTGCCATCTTT         480
            x         x         x         x         x         x

  161   AlaLysHisArgArgSerProGlyGluArgPheLeuCysGlyGlyIleLeuIleSerSer         180
  481   GCCAAGCACAGGAGGTCGCCCGGAGAGCGGTTCCTGTGCGGGGGCATACTCATCAGCTCC         540
            x         x         x         x         x         x

  181   CysTrpIleLeuSerAlaAlaHisCysPheGlnGluArgPheProProHisHisLeuThr         200
  541   TGCTGGATTCTCTCTGCCGCCCACTGCTTCCAGGAGAGGTTTCCGCCCCACCACCTGACG         600
            x         x         x         x         x         x

  201   ValIleLeuGlyArgThrTyrArgValValProGlyGluGluGluGlnLysPheGluVal         220
  601   GTGATCTTGGGCAGAACATACCGGGTGGTCCCTGGCGAGGAGGAGCAGAAATTTGAAGTC         660
            x         x         x         x         x         x

  221   GluLysTyrIleValHisLysGluPheAspAspAspThrTyrAspAsnAspIleAlaLeu         240
  661   GAAAAATACATTGTCCATAAGGAATTCGATGATGACACTTACGACAATGACATTGCGCTG         720
            x         x         x         x         x         x

  241   LeuGlnLeuLysSerAspSerSerArgCysAlaGlnGluSerSerValValArgThrVal         260
  721   CTGCAGCTGAAATCGGATTCGTCCCGCTGTGCCCAGGAGAGCAGCGTGGTCCGCACTGTC         780
            x         x         x         x         x         x

  261   CysLeuProProAlaAspLeuGlnLeuProAspTrpThrGlyCysGluLeuSerGlyTyr         280
  781   TGCCTTCCCCCGGCGGACCTGCAGCTGCCGGACTGGACGGGTGTGAGCTCTCCGGCTAC         840
            x         x         x         x         x         x

  281   GlyLysHisGluAlaLeuSerProPheTyrSerGluArgLeuLysGluAlaHisValArg         300
  841   GGCAAGCATGAGGCCTTGTCTCCTTTCTATTCGGAGCGGCTGAAGGAGGCTCATGTCAGA         900
            x         x         x         x         x         x

  301   LeuTyrProSerSerArgCysThrSerGlnHisLeuLeuAsnArgThrValThrAspAsn         320
  901   CTGTACCCATCCAGCCGCTGCACCATCACAACATTTACTTAACAGAACAGTCACCGACAAC         960
            x         x         x         x         x         x

  321   MetLeuCysAlaGlyAspThrArgSerGlyGlyProGlnAlaAsnLeuHisAspAlaCys         340
  961   ATGCTGTGTGCTGGAGACACTCGGAGCGGCGGGCCCCAGGCAAACTTGCACGACGCCTGC        1020
            x         x         x         x         x         x

  341   GlnGlyAspSerGlyGlyProLeuValCysLeuAsnAspGlyArgMetThrLeuValGly         360
 1021   CAGGGCGATTCGGGAGGCCCCCTGGTGTGTCTGAACGATGGCCGCATGACTTTGGTCGGC        1080
            x         x         x         x         x         x

  361   IleIleSerTrpGlyLeuGlyCysGlyGlnLysAsnValProGlyValTyrThrLysVal         380
 1081   ATCATCAGCTGGGGCCTGGGCTGTGGACAGAAGAATGTCCCCGGTGTCTACACAAAGGTT        1140
            x         x         x         x         x         x

  381   ThrAsnTyrLeuAspTrpIleArgAspAsnMetArgPro                             400
 1141   ACCAACTACCTAGACTGGATTCGTGACAACATGCGGACCG                           1200
            x         x         x         x
```

FIG.3

**EUROPÄISCHER RECHERCHENBERICHT**

Europäisches Patentamt

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP-A-0 093 619 (GENENTECH INC.) * Seite 9, Zeilen 19-33; Ansprüche * | 1,9 | C 12 N 15/00<br>A 61 K 37/54<br>C 12 N 9/64 |
| | --- | | |
| Y | PROCEEDINGS OF THE NATIONAL ACADEMY OF SCIENCES OF THE USA, Band 81, Nr. 17, September 1984, Seiten 5355-5359, Wahington, US; T. NY et al.: "The structure of the human tissue-type plasminogen activator gene: Correlation of intron and exon structures to functional and structural domains" * Zusammenfassung; Seite 5355, Spalte 1, Zeilen 18-39; Seite 5358, Spalte 1, Zeile 1 - Seite 5359, Spalte 1, Zeile 28; Figur 4 * | 1,9 | |
| | --- | | RECHERCHIERTE SACHGEBIETE (Int. Cl 4) |
| P,X | PROCEEDINGS OF THE NATIONAL ACADEMY OF SCIENCES OF THE USA, Band 83, Nr. 13, Juli 1986, Seiten 4670-4674, Washington, US; A.-J. VAN ZONNEVELD et al.: "Autonomous functions of structural domains on human tissue-type plasminogen activator" * Zusammenfassung; Figur 1; Diskussion * | 1 | C 12 N |
| | ---     -/- | | |

Der vorliegende Recherchenbericht wurde für alle Patentansprüche erstellt.

| Recherchenort | Abschlußdatum der Recherche | Prüfer |
|---|---|---|
| DEN HAAG | 04-06-1987 | YEATS S.M. |

**Europäisches Patentamt**

## EUROPÄISCHER RECHERCHENBERICHT

EP  87 10 5806

Seite 2

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 103, Nr. 21 25. November 1985, Seite 219, Zusammenfassung Nr. 173375z, Columbus, Ohio, US; H. KAGITANI et al.: "Expression in E. coli of finger-domain-lacking tissue-type plasminogen activator with high fibrin affinity", & FEBS LETT. 1985, 189(1), 145-9 <br> * Zusammenfassung * <br><br> --- | 1,9 | |
| P,X | EP-A-0 207 589  (BEECHAM GROUP PLC) <br><br> * Seite 7, Zeile 42  -  Seite  8, Zeile 50; Seite 16, Zeilen 20-36; Ansprüche * <br><br> ----- | 1-4,8, 13,14, 29,32 | RECHERCHIERTE SACHGEBIETE (Int. Cl 4) |

Der vorliegende Recherchenbericht wurde für alle Patentansprüche erstellt.

| Recherchenort | Abschlußdatum der Recherche | Prüfer |
|---|---|---|
| DEN HAAG | 04-06-1987 | YEATS S.M. |